# EUROPÄISCHE PATENTSCHRIFT

(11) **EP 2 259 779 B1**
(45) Veröffentlichungstag und Bekanntmachung des Hinweises auf die Patenterteilung: **16.09.2015**
(21) Anmeldenummer: 09718165.5
(22) Anmeldetag: 04.02.2009
(51) Int. Cl.: A61K 9/20

(54) **VERBESSERTE ISOMALT-HALTIGE KOMPRIMATE UND VERFAHREN ZU IHRER HERSTELLUNG**
IMPROVED ISOMALT-CONTAINING TABLETS AND METHODS FOR THE PRODUCTION THEREOF
COMPRIMÉS AMÉLIORÉS CONTENANT DE L'ISOMALT ET LEURS PROCÉDÉS DE FABRICATION

(30) Priorität: 01.03.2008 DE 102008012015
(43) Veröffentlichungstag der Anmeldung: 15.12.2010
(73) Patentinhaber: Südzucker Aktiengesellschaft Mannheim/Ochsenfurt, 68165 Mannheim (DE)
(72) Erfinder: KOWALCZYK, Jörg, 67304 Eisenberg/Steinborn (DE); LUHN, Oliver, 67134 Birkenheide (DE)
(74) Vertreter: Schwahn, Hartmut
(86) Internationale Anmeldenummer: PCT/EP2009/000712
(87) Internationale Veröffentlichungsnummer: WO 2009/109266

(56) Entgegenhaltungen:
- WO-A-02/076211
- DE-A1- 19 943 491
- BORDE B ET AL: "Thermal properties of isomalt: A diastereomer mixture" JOURNAL OF THERMAL ANALYSIS AND CALORIMETRY, KLUWER ACADEMIC PUBLISHERS, DO, Bd. 69, Nr. 1, 1. Juli 2002 (2002-07-01), Seiten 267-280, XP019254220 ISSN: 1572-8943

## Beschreibung

Die vorliegende Erfindung betrifft verbesserte Isomalt-haltige Komprimate und Verfahren zu deren Herstellung.

Bei der Entwicklung pharmazeutischer Darreichungsformen wird der Bioverfügbarkeit des Wirkstoffs in höchstem Maße Beachtung geschenkt. Komprimate, auch als Tabletten bekannt, werden häufig zur Verabreichung von pharmazeutisch aktiven Wirkstoffen verwendet. Um diese Wirkstoffe gezielt freizusetzen werden in der Galenik Komprimate unterschiedlicher Zusammensetzung und Verarbeitung bereitgestellt, die eine spezifische Auflösedauer, also Zerfallszeit, aufweisen.

Schnellzerfallende Komprimate, sogenannte "Oro-Dispersible Tablets" (ODT), gewinnen im Markt zunehmend an Bedeutung. Bei dieser Darreichungsform ist eine Zerfallszeit im Mundraum von weniger als 60 Sekunden, insbesondere weniger als 20 Sekunden erwünscht, ohne dass eine zusätzliche Flüssigkeitsaufnahme erforderlich ist.

Die Verwendung von Gemischen aus 1,6-GPS (6-O-α-D-Glucopyranosyl-D-sorbit) und 1,1-GPM (1-O-α-D-Glucopyranosyl-D-mannit) zur Herstellung von Komprimaten ist aus der DE 196 39 343 A1 und der DE 199 43 491 A1 bekannt.

Isomalt ist ein Gemisch der beiden Stereoisomere 1,6-GPS und 1,1-GPM. Isomalt ist auch als Palatinit^{®} oder galenlQ^{®} bekannt. Isomalt wird durch die Hydrierung von Isomaltulose gewonnen. In Arzneibüchern wird Isomalt als zu mindestens 98 % reine Mischung aus 1,6-GPS und 1,1-GPM definiert, wobei keine der beiden Komponenten zu weniger als 3 %, bezogen auf die Trockensubstanz, enthalten ist.

Aufgrund der sensorischen Eigenschaften von Isomalt, ergibt sich grundsätzlich eine Eignung für die Verwendung in Komprimaten.

Ein der vorliegenden Erfindung zugrunde liegendes technisches Problem ist die Bereitstellung von Isomalt-haltigen Komprimaten mit verbesserter Komprimathärte bei gleicher Presskraft. Insbesondere ist ein der Erfindung zugrunde liegendes Technische Problem die Bereitstellung von Isomalt-haltigen Komprimaten mit verringerter Komprimathärte bei gleicher Presskraft.

Ein anderes der vorliegenden Erfindung zugrunde liegendes technisches Problem ist die Bereitstellung von Isomalt-haltigen Komprimaten mit verbesserter Auflösedauer bei gleicher Presskraft. Insbesondere ist ein der Erfindung zugrunde liegendes Technische Problem die Bereitstellung von Isomalt-haltigen Komprimaten mit verringerter Auflösedauer bei gleicher Presskraft.

Ein anderes der vorliegenden Erfindung zugrunde liegendes technisches Problem ist die Bereitstellung von Verfahren zur Regulierung der Komprimathärte bei Isomalt-haltigen Komprimaten. Insbesondere ist ein der Erfindung zugrunde liegendes Technische Problem die Bereitstellung von Verfahren zur Verringerung der Komprimathärte bei Isomalt-haltigen Komprimaten.

Ein anderes der vorliegenden Erfindung zugrunde liegendes technisches Problem ist die Bereitstellung von Verfahren zur Regulierung der Auflösedauer bei Isomalt-haltigen Komprimaten. Insbesondere ist ein der Erfindung zugrunde liegendes Technische Problem die Bereitstellung von Verfahren zur Verringerung der Auflösedauer bei Isomalt-haltigen Komprimaten.

Ein anderes der vorliegenden Erfindung zugrunde liegendes technisches Problem ist die Bereitstellung von Verfahren zur Herstellung von Isomalt-haltigen Komprimaten, die einen geringen Anteil an Magnesiumstearat enthalten, insbesondere von Isomalt-haltigen Komprimaten, die kein Magnesiumstearat enthalten, sowie von solchen Isomalt-haltigen Komprimaten selbst.

Die Erfindung löst die ihr zugrunde liegenden technischen Probleme durch die Bereitstellung von Verfahren und Komprimaten gemäß der Ansprüche.

Insbesondere löst die Erfindung die ihr zugrunde liegenden technischen Probleme durch die Bereitstellung eines Verfahrens zur Herstellung eines Komprimates, wobei Isomalt mit 1,6-GPS (6-O-α-D-Glucopyranosyl-D-sorbit) oder mit 1,1-GPM (1-O-α-D-Glucopyranosyl-D-mannit) vermischt wird und die Mischung zu einem Komprimat verpresst wird.

Im Zusammenhang mit dieser Erfindung wird unter Isomalt eine Zusammensetzung aus 1,6-GPS und 1,1-GPM verstanden.

Erfindungsgemäß bevorzugt enthält das Isomalt mindestens 98 Gew.-% 1,6-GPS und 1,1-GPM. Erfindungsgemäß bevorzugt enthält das Isomalt mindestens 3 Gew.-% 1,6-GPS und mindestens 3 Gew.-% 1,1-GPM. Erfindungsgemäß bevorzugt enthält das Isomalt mindestens 98 Gew.-% 1,6-GPS und 1,1-GPM und mindestens 3 Gew.-% 1,6-GPS und mindestens 3 Gew.-% 1,1-GPM.

Erfindungsgemäß bevorzugt enthält das Isomalt 20 Gew.-% bis 90 Gew.-% 1,6-GPS. Erfindungsgemäß bevorzugt enthält das Isomalt 40 Gew.-% bis 85 Gew.-% 1,6-GPS. Erfindungsgemäß bevorzugt enthält das Isomalt 43 Gew.-% bis 80 Gew.-% 1,6-GPS. Erfindungsgemäß bevorzugt enthält das Isomalt 43 Gew.-% bis 57 Gew.-% 1,6-GPS. Erfindungsgemäß bevorzugt enthält das Isomalt 75 Gew.-% bis 80 Gew.-% 1,6-GPS.

Erfindungsgemäß bevorzugt enthält das Isomalt 15 Gew.-% bis 90 Gew.-% 1,1-GPM. Erfindungsgemäß bevorzugt enthält das Isomalt 40 Gew.-% bis 85 Gew.-% 1,1-GPM. Erfindungsgemäß bevorzugt enthält das Isomalt 43 Gew.-% bis 60 Gew.-% 1,1-GPM. Erfindungsgemäß bevorzugt enthält das Isomalt 43 Gew.-% bis 57 Gew.-% 1,1-GPM. Erfindungsgemäß bevorzugt enthält das Isomalt 75 Gew.-% bis 80 Gew.-% 1,1-GPM.

Erfindungsgemäß bevorzugt enthält das Isomalt 1,6-GPS und 1,1-GPM in einem Verhältnis von etwa 1 zu 1, besonders bevorzugt in einem Verhältnis von 1 zu 1. Erfindungsgemäß bevorzugt enthält das Isomalt 1,6-GPS und 1,1-GPM in einem Verhältnis von etwa 3 zu 1, besonders bevorzugt in einem Verhältnis von 3 zu 1.

Im Stand der Technik wird Isomalt häufig aus der Hydrierung, insbesondere katalytische Hydrierung, von Isomaltulose gewonnen, wobei ein nahezu äquimolares Gemisch aus 1,6-GPS und 1,1-GPM erhalten wird. Das gewünschte Verhältnis von 1,6-GPS und 1,1-GPM im Isomalt kann dann durch dem Fachmann bekannte Verfahren, insbesondere Anreicherungsverfahren, wie beispielsweise Fällen oder Auskristallisieren, gewonnen werden.

Erfindungsgemäß bevorzugt wird das Isomalt aus der Hydrierung von Isomattulose gewonnen. Erfindungsgemäß bevorzugt wird das Verhältnis von 1,6-GPS und 1,1-GPM im Isomalt zwischen 1 zu 99 und 99 zu 1, besonders bevorzugt zwischen 3 zu 97 und 97 zu 3, ganz besonders bevorzugt zwischen 1 zu 9 und 9 zu 1 eingestellt.

Erfindungsgemäß bevorzugt hat mindestens 95 Gew.-% des Isomalts eine Korngröße von höchstens 2000 µm, besonders bevorzugt 1000 µm. Erfindungsgemäß bevorzugt hat mindestens 95 Gew.-% des Isomalts eine Korngröße von höchstens 500 µm.

Erfindungsgemäß bevorzugt hat das Isomalt eine Korngröße von höchstens 2000 µm, besonders bevorzugt 1000 µm. Erfindungsgemäß bevorzugt hat das Isomalt eine Korngröße von höchstens 750 µm, besonders bevorzugt 600 µm.

Erfindungsgemäß bevorzugt hat 10 Gew.-% bis 90 Gew.-% des I-somalts eine Korngröße von mindestens 150 µm, besonders bevorzugt von mindestens 200 µm, ganz besonders bevorzugt von mindestens 250 µm. Erfindungsgemäß bevorzugt hat 35 Gew.-% bis 70 - Gew.-% des Isomalts eine Korngröße von mindestens 150 µm, besonders bevorzugt von mindestens 200 µm, ganz besonders bevorzugt von mindestens 250 µm. Erfindungsgemäß bevorzugt hat mindestens 10 Gew.-% des Isomalts eine Korngröße von mindestens 150 µm, besonders bevorzugt von mindestens 200 µm, ganz besonders bevorzugt von mindestens 250 µm. Erfindungsgemäß bevorzugt hat mindestens 20 Gew.-% des Isomalts eine Korngröße von mindestens 150 µm, besonders bevorzugt von mindestens 200 µm, ganz besonders bevorzugt von mindestens 250 µm. Erfindungsgemäß bevorzugt hat mindestens 30 Gew.-% des Isomalts eine Korngröße von mindestens 150 µm, besonders bevorzugt von mindestens 200 µm, ganz besonders bevorzugt von mindestens 250 µm. Erfindungsgemäß bevorzugt hat mindestens 35 Gew.-% des Isomalts eine Korngröße von mindestens 150 µm, besonders bevorzugt von mindestens 200 µm, ganz besonders bevorzugt von mindestens 250 µm. Erfindungsgemäß bevorzugt hat mindestens 40 Gew.-% des Isomalts eine Korngröße von mindestens 150 µm, besonders bevorzugt von mindestens 200 µm, ganz besonders bevorzugt von mindestens 250 µm. Erfindungsgemäß bevorzugt hat höchstens 90 Gew.-% des Isomalts eine Korngröße von mindestens 150 µm, besonders bevorzugt von mindestens 200 µm, ganz besonders bevorzugt von mindestens 250 µm. Erfindungsgemäß bevorzugt hat höchstens 80 Gew.-% des Isomalts eine Korngröße von mindestens 150 µm, besonders bevorzugt von mindestens 200 µm, ganz besonders bevorzugt von mindestens 250 µm. Erfindungsgemäß bevorzugt hat höchstens 70 Gew.-% des Isomalts eine Korngröße von mindestens 150 µm, besonders bevorzugt von mindestens 200 µm, ganz besonders bevorzugt von mindestens 250 µm.

Erfindungsgemäß bevorzugt hat mindestens 80 Gew.-% des Isomalts eine Korngröße von mindestens 50 µm, besonders bevorzugt 60 µm. Erfindungsgemäß bevorzugt hat mindestens 80 Gew.-% des Isomalts eine Korngröße von mindestens 63 µm.

Erfindungsgemäß bevorzugt hat mindestens 85 Gew.-% des Isomalts eine Korngröße von mindestens 50 µm, besonders bevorzugt 60 µm. Erfindungsgemäß bevorzugt hat mindestens 85 Gew.-% des Isomalts eine Korngröße von mindestens 63 µm. Erfindungsgemäß bevorzugt hat mindestens 90 Gew.-% des Isomalts eine Korngröße von mindestens 50 µm, besonders bevorzugt 60 µm. Erfindungsgemäß bevorzugt hat mindestens 90 Gew.-% des Isomalts eine Korngröße von mindestens 63 µm.

Erfindungsgemäß bevorzugt hat das Isomalt eine Korngröße von mindestens 10 µm, besonders bevorzugt 50 µm. Erfindungsgemäß bevorzugt hat das Isomalt eine Korngröße von höchstens 60 µm, besonders bevorzugt 63 µm.

Erfindungsgemäß bevorzugt wird die Korngröße durch Siebmessung, besonders bevorzugt durch einen mechanischen Sieb-Schüttler bestimmt. Erfindungsgemäß bevorzugt wird die Korngröße nach dem Verfahren gemäß Ph. Eur. 2.9.12 des Europäischen Arzneibuchs bestimmt.

Erfindungsgemäß bevorzugt handelt es sich bei dem Isomalt um agglomeriertes Isomalt. Erfindungsgemäß bevorzugt wurde das Isomalt vor der Verwendung in dem erfindungsgemäßen Verfahren agglomeriert.

Erfindungsgemäß bevorzugt handelt es sich bei dem Isomalt um nicht agglomeriertes Isomalt.

Erfindungsgemäß bevorzugt handelt es sich bei dem Isomalt um galenlQ720^{®}. Erfindungsgemäß bevorzugt handelt es sich bei dem Isomalt um galenlQ721^{®}.

Die Komprimathärte und die Auflösedauer von Komprimaten hängen unter anderem von der Art und dem Ausmaß der Partikeldeformation beim Pressvorgang ab. Art und Ausmaß der Partikeldeformation beim Pressvorgang hängen wiederum von den mechanischen Materialeigenschaften, insbesondere dem E-Modul, von der Größe der Partikel, von der Form der Partikel, zum Beispiel der Kristallform, von der Isotropie der Partikel, beispielsweise Fehler im Kristallaufbau, und von der Lage zur angreifenden Kraft ab.

Es zeigte sich überraschender Weise, dass sich die Komprimathärte und Auflösedauer eines Isomalt-haltigen Komprimats ändert, wenn das Isomalt vor dem Verpressen mit zusätzlichem 1,6-GPS oder 1,1-GPM vermischt wird. Dabei wurde überraschender Weise festgestellt, dass das Verpressen von Isomalt mit einem bestimmten Verhältnis von 1,6-GPS und 1,1-GPM zu einem Komprimat mit anderer Komprimathärte und/oder Auflösedauer führt als das gleichartige Verpressen mit gleicher Anpresskraft von einem Gemisch aus Isomalt und 1,6-GPS oder 1,1-GPM, das insgesamt das gleiche Verhältnis von 1,6-GPS und 1,1-GPM aufweist. Insbesondere wurde überraschender Weise gefunden, dass sich bei einem Isomalt-haltigen Komprimat die Komprimathärte verringern und die Auflösedauer verkürzen lässt, in dem das Isomalt vor dem Verpressen mit 1,-6-GPS oder 1,1-GPM vermischt wird. Ohne an die Theorie gebunden zu sein, könnten dabei der unterschiedliche Aufbau des im Isomalt enthaltenen 1,6-GPS und 1,1-GPM und des zusätzlichen 1,6-GPS oder 1,1-GPM für die Veränderung der Komprimathärte und der Auflösedauer verantwortlich sein. Beispielsweise könnte das Vorkommen von Tomahawk-Strukturen in dem zusätzlich vermischten 1,6-GPS oder 1,1-GPM zu einem veränderten Gesamtgefüge in dem Komprimat führen. Auch könnte eine andere Verteilung der Kristalle die Komprimathärte und die Auflösedauer beeinflussen. Ohne an die Theorie gebunden zu sein, könnte Isomalt in Mosaik-kristall-Strukturen vorliegen, während das zugegebene 1,6-GPS oder 1,1-GPM keine Mosaik-Kristall-Strukturen aufweist. Dadurch könnten sich Komprimate, die erfindungsgemäß hergestellt wurden, bei vergleichbarer Korngröße durch ihre spezifische Oberfläche von Isomalt-Komprimaten aus dem Stand der Technik unterscheiden. Auch könnten die unterschiedlichen Kristallstrukturen in den Komprimaten zu Unterschieden im Gefügeaufbau, insbesondere zu unterschiedlichen Porendurchmessern und/oder Porengrtißenverteilungen führen.

Im Zusammenhang mit der vorliegenden Erfindung wird unter einem Komprimat eine Tablette verstanden, die unter Pressdruck aus Pulvern und/oder Granulaten gefertigt wird. Das verpresste Komprimat hat eine bestimmte Komprimathärte.

Die Komprimathärte kann zum Beispiel nach dem Verfahren nach Ph. Eur. 2.9.8 des Europäischen Arzneibuchs bestimmt werden. Beispielsweise kann als Messprinzip eine Kraftmessdose mit Dehnungsmessstreifen verwendet werden. Beispielsweise kann als Messgerät ein Gerät TBH 30 der Firma Erweka verwendet werden.

Das Verpressen mit einem bestimmten Pressdruck ist dem Fachmann bekannt. Das Verpressen kann mit aus dem Stand der Technik bekannten Methoden erfolgen.

Erfindungsgemäß bevorzugt wird die Mischung aus Isomalt und 1,6-GPS oder 1,1-GPM direkt verpresst. Erfindungsgemäß bevorzugt wird die Mischung aus Isomalt und 1,6-GPS direkt verpresst. Erfindungsgemäß bevorzugt wird die Mischung aus Isomalt und 1,1-GPM direkt verpresst.

Erfindungsgemäß bevorzugt wird mit einer Kraft von 1,0 bis 15,0 kN verpresst. Erfindungsgemäß bevorzugt wird mit einer Kraft von 2,0 bis 12,0 kN verpresst. Erfindungsgemäß bevorzugt wird mit einer Kraft von 2,0 bis 10,0 kN verpresst. Erfindungsgemäß bevorzugt wird mit einer Kraft von 2,0 bis 8,0 kN verpresst. Erfindungsgemäß bevorzugt wird mit einer Kraft von mindestens 1,0 kN verpresst. Erfindungsgemäß bevorzugt wird mit einer Kraft von mindestens 2,0 kN verpresst. Erfindungsgemäß bevorzugt wird mit einer Kraft von mindestens 3,0 kN verpresst. Erfindungsgemäß bevorzugt wird mit einer Kraft von mindestens 4,0 kN verpresst. Erfindungsgemäß bevorzugt wird mit einer Kraft von höchstens 20,0 kN verpresst. Erfindungsgemäß bevorzugt wird mit einer Kraft von höchstens 15,0 kN verpresst. Erfindungsgemäß bevorzugt wird mit einer Kraft von höchstens 12,0 kN verpresst. Erfindungsgemäß bevorzugt wird mit einer Kraft von höchstens 10,0 kN verpresst. Erfindungsgemäß bevorzugt wird mit einer Kraft von höchstens 8,0 kN verpresst.

Im Zusammenhang mit der vorliegenden Erfindung wird unter dem 1,6-GPS und dem 1,1-GPM, das mit dem Isomalt vermischt wird, solches 1,6-GPS und 1,1-GPM verstanden, das in dem verwendeten Isomalt vor dem Mischen nicht enthalten ist. Bei dem 1,6-GPS und dem 1,1-GPM, das mit dem Isomalt vermischt wird handelt es sich also nicht um das in der Isomaltfraktion enthaltene1,6-GPS und 1,1-GPM. Erfindungsgemäß wird also das als Isomalt vorhandene 1,6-GPS und 1,1-GPM mit zusätzlichem 1,6-GPS oder mit zusätzlichem 1,1-GPM vermischt.

Erfindungsgemäß bevorzugt wird das 1,6-GPS oder 1,1-GPM mit dem Isomalt physikalisch vermischt. Das Vermischen kann beispielsweise mittels eines Diffusionsmischer geschehen. Erfindungsgemäß bevorzugt ist das mit dem Isomalt vermischte 1,6-GPS oder 1,1-GPM nicht mit dem Isomalt agglomeriert.

Erfindungsgemäß bevorzugt wird Isomalt mit 1,6-GPS vermischt und die Mischung zu einem Komprimat verpresst. Erfindungsgemäß bevorzugt wird Isomalt mit 1,1-GPM vermischt und die Mischung zu einem Komprimat verpresst.

Erfindungsgemäß bevorzugt beträgt das Verhältnis zwischen Isomalt und 1,6-GPS oder 1,1-GPM 1 : 99 bis 99 : 1. Erfindungsgemäß bevorzugt beträgt das Verhältnis zwischen Isomalt und 1,6-GPS oder 1,1-GPM 1 : 9 bis 9 : 1. Erfindungsgemäß bevorzugt beträgt das Verhältnis zwischen Isomalt und 1,6-GPS oder 1,1-GPM 2 : 8 bis 8 : 2. Erfindungsgemäß bevorzugt beträgt das Verhältnis zwischen Isomalt und 1,6-GPS oder 1,1-GPM 3 : 7 bis 7 : 3. Erfindungsgemäß bevorzugt beträgt das Verhältnis zwischen Isomalt und 1,6-GPS oder 1,1-GPM 4 : 6 bis 6 : 4. Erfindungsgemäß bevorzugt beträgt das Verhältnis zwischen Isomalt und 1,6-GPS oder 1,1-GPM 1 : 1 bis 7 : 3. Erfindungsgemäß bevorzugt beträgt das Verhältnis zwischen Isomalt und 1,6-GPS oder 1,1-GPM 1 : 1 bis 6 : 4. Erfindungsgemäß bevorzugt beträgt das Verhältnis zwischen Isomalt und 1,6-GPS oder 1,1-GPM 1 : 1 bis 4 : 1. Erfindungsgemäß bevorzugt beträgt das Verhältnis zwischen Isomalt und 1,6-GPS oder 1,1-GPM 1 : 1 bis 9 : 1. Erfindungsgemäß bevorzugt beträgt das Verhältnis zwischen Isomalt und 1,6-GPS oder 1,1-GPM 1 : 1. Erfindungsgemäß bevorzugt beträgt das Verhältnis zwischen Isomalt und 1,6-GPS oder 1,1-GPM 7 : 3.

Erfindungsgemäß bevorzugt beträgt das Verhältnis zwischen Isomalt und 1,6-GPS 1 : 99 bis 99 : 1. Erfindungsgemäß bevorzugt beträgt das Verhältnis zwischen Isomalt und 1,6-GPS 1 : 9 bis 9 : 1. Erfindungsgemäß bevorzugt beträgt das Verhältnis zwischen Isomalt und 1,6-GPS 2 : 8 bis 8 : 2. Erfindungsgemäß bevorzugt beträgt das Verhältnis zwischen Isomalt und 1,6-GPS 3 : 7 bis 7 : 3. Erfindungsgemäß bevorzugt beträgt das Verhältnis zwischen Isomalt und 1,6-GPS 4 : 6 bis 6 : 4. Erfindungsgemäß bevorzugt beträgt das Verhältnis zwischen Isomalt und 1,6-GPS 1 : 1 bis 7 : 3. Erfindungsgemäß bevorzugt beträgt das Verhältnis zwischen Isomalt und 1,6-GPS 1 : 1 bis 6 : 4. Erfindungsgemäß bevorzugt beträgt das Verhältnis zwischen Isomalt und 1,6-GPS 1 : 1 bis 4 : 1. Erfindungsgemäß bevorzugt beträgt das Verhältnis zwischen Isomalt und 1,6-GPS 1 : 1 bis 9 : 1. Erfindungsgemäß bevorzugt beträgt das Verhältnis zwischen Isomalt und 1,6-GPS 1 : 1. Erfindungsgemäß bevorzugt beträgt das Verhältnis zwischen Isomalt und 1,6-GPS 7 : 3.

Erfindungsgemäß bevorzugt beträgt das Verhältnis zwischen Isomalt und 1,1-GPM 1 : 99 bis 99 : 1. Erfindungsgemäß bevorzugt beträgt das Verhältnis zwischen Isomalt 1,1-GPM 1 : 9 bis 9 : 1. Erfindungsgemäß bevorzugt beträgt das Verhältnis zwischen Isomalt und 1,1-GPM 2 : 8 bis 8 : 2. Erfindungsgemäß bevorzugt beträgt das Verhältnis zwischen Isomalt und 1,1-GPM 3 : 7 bis 7 : 3. Erfindungsgemäß bevorzugt beträgt das Verhältnis zwischen Isomalt und 1,1-GPM 4 : 6 bis 6 : 4. Erfindungsgemäß bevorzugt beträgt das Verhältnis zwischen Isomalt und 1,1-GPM 1 : 1 bis 7 : 3. Erfindungsgemäß bevorzugt beträgt das Verhältnis zwischen Isomalt und 1,1-GPM 1 : 1 bis 6 : 4. Erfindungsgemäß bevorzugt beträgt das Verhältnis zwischen Isomalt und 1,1-GPM 1 : 1 bis 4 : 1. Erfindungsgemäß bevorzugt beträgt das Verhältnis zwischen Isomalt und 1,1-GPM 1 : 1 bis 9 : 1. Erfindungsgemäß bevorzugt beträgt das Verhältnis zwischen Isomalt und 1,1-GPM 1 : 1. Erfindungsgemäß bevorzugt beträgt das Verhältnis zwischen Isomalt und 1,1-GPM 7 : 3.

Erfindungsgemäß bevorzugt wird das 1,6-GPS oder das 1,1-GPM vor dem Vermischen mit dem Isomalt gemahlen. Erfindungsgemäß bevorzugt wird das 1,6-GPS vor dem Vermischen mit dem Isomalt gemahlen. Erfindungsgemäß bevorzugt wird das 1,1-GPM vor dem Vermischen mit dem Isomalt gemahlen.

Erfindungsgemäß bevorzugt werden das 1,6-GPS oder das 1,1-GPM vor dem Vermischen mit dem Isomalt gesiebt. Erfindungsgemäß bevorzugt wird das 1,6-GPS vor dem Vermischen mit dem Isomalt gesiebt. Erfindungsgemäß bevorzugt wird das 1,1-GPM vor dem Vermischen mit dem Isomalt gesiebt.

Erfindungsgemäß bevorzugt sind das 1,6-GPS oder das 1,1-GPM vor dem Vermischen mit dem Isomalt nicht agglomeriert.. Erfindungsgemäß bevorzugt ist das 1,6-GPS vor dem Vermischen mit dem Isomalt nicht agglomeriert. Erfindungsgemäß bevorzugt ist das 1,1-GPM vor dem Vermischen mit dem Isomalt nicht agglomeriert.

Erfindungsgemäß bevorzugt hat das 1,6-GPS oder das 1,1-GPM vor dem Vermischen mit dem Isomalt eine Tomahawk-Struktur. Erfindungsgemäß bevorzugt hat das 1,6-GPS vor dem Vermischen mit dem Isomalt eine Tomahawk-Struktur. Erfindungsgemäß bevorzugt hat das 1,1-GPM vor dem Vermischen mit dem Isomalt eine Tomahawk-Struktur.

Erfindungsgemäß bevorzugt hat das 1,6-GPS oder das 1,1-GPM vor dem Vermischen mit dem Isomalt eine Partikelgröße von höchstens 500 µm. Erfindungsgemäß bevorzugt hat das 1,6-GPS oder das 1,1-GPM vor dem Vermischen mit dem Isomalt eine Partikelgröße von höchstens 250 µm. Erfindungsgemäß bevorzugt hat das 1,6-GPS oder das 1,1-GPM vor dem Vermischen mit dem Isomalt eine Partikelgröße von höchstens 150 µm. Erfindungsgemäß bevorzugt hat das 1,6-GPS oder das 1,1-GPM vor dem Vermischen mit dem Isomalt eine Partikelgröße von höchstens 100 µm. Erfindungsgemäß bevorzugt hat das 1,6-GPS oder das 1,1-GPM vor dem Vermischen mit dem Isomalt eine Partikelgröße von 40 µm bis 150 µm. Erfindungsgemäß bevorzugt hat das 1,6-GPS oder das 1,1-GPM vor dem Vermischen mit dem Isomalt eine Partikelgröße von 50 µm bis 100 µm. Erfindungsgemäß bevorzugt hat das 1,6-GPS oder das 1,1-GPM vor dem Vermischen mit dem Isomalt eine Partikelgröße von 63 µm bis 90 µm. Erfindungsgemäß bevorzugt hat das 1,6-GPS oder das 1,1-GPM vor dem Vermischen mit dem Isomalt eine Partikelgröße von mindestens 0,2 µpm. Erfindungsgemäß bevorzugt hat das 1,6-GPS oder das 1,1-GPM vor dem Vermischen mit dem Isomalt eine Partikelgröße von mindestens 1 µm. Erfindungsgemäß bevorzugt hat das 1,6-GPS oder das 1,1-GPM vor dem Vermischen mit dem Isomalt eine Partikelgröße von mindestens 10 µm. Erfindungsgemäß bevorzugt hat das 1,6-GPS oder das 1,1-GPM vor dem Vermischen mit dem Isomalt eine Partikelgröße von mindestens 20 µm. Erfindungsgemäß bevorzugt hat das 1,6-GPS oder das 1,1-GPM vor dem Vermischen mit dem Isomalt eine Partikelgröße von mindestens 30 µm. Erfindungsgemäß bevorzugt hat das 1,6-GPS oder das 1,1-GPM vor dem Vermischen mit dem Isomalt eine Partikelgröße von mindestens 40 µm. Erfindungsgemäß bevorzugt hat das 1,6-GPS oder das 1,1-GPM vor dem Vermischen mit dem Isomalt eine Partikelgröße von mindestens 50 µm. Erfindungsgemäß bevorzugt hat das 1,6-GPS oder das 1,1-GPM vor dem Vermischen mit dem Isomalt eine Partikelgröße von mindestens 60 µm. Erfindungsgemäß bevorzugt hat das 1,6-GPS oder das 1,1-GPM vor dem Vermischen mit dem Isomalt eine Partikelgröße von mindestens 63 µm.

Erfindungsgemäß bevorzugt hat das 1,6-GPS vor dem Vermischen mit dem Isomalt eine Partikelgröße von höchstens 500 µm. Erfindungsgemäß bevorzugt hat das 1,6-GPS vor dem Vermischen mit dem Isomalt eine Partikelgröße von höchstens 250 µm. Erfindungsgemäß bevorzugt hat das 1,6-GPS vor dem Vermischen mit dem Isomalt eine Partikelgröße von höchstens 150 µm. Erfindungsgemäß bevorzugt hat das 1,6-GPS vor dem Vermischen mit dem Isomalt eine Partikelgröße von höchstens 100 µm. Erfindungsgemäß bevorzugt hat das 1,6-GPS vor dem Vermischen mit dem Isomalt eine Partikelgröße von 40 µm bis 150 µm. Erfindungsgemäß bevorzugt hat das 1,6-GPS vor dem Vermischen mit dem Isomalt eine Partikelgröße von 50 µm bis 100 µm. Erfindungsgemäß bevorzugt hat das 1,6-GPS vor dem Vermischen mit dem Isomalt eine Partikelgröße von 63 µm bis 90 µm. Erfindungsgemäß bevorzugt hat das 1,6-GPS vor dem Vermischen mit dem Isomalt eine Partikelgröße von mindestens 0,2 µm. Erfindungsgemäß bevorzugt hat das 1,6-GPS vor dem Vermischen mit dem Isomalt eine Partikelgröße von mindestens 1 µm. Erfindungsgemäß bevorzugt hat das 1,6-GPS vor dem Vermischen mit dem Isomalt eine Partikelgröße von mindestens 10 µm. Erfindungsgemäß bevorzugt hat das 1,6-GPS vor dem Vermischen mit dem Isomalt eine Partikelgröße von mindestens 20 µm. Erfindungsgemäß bevorzugt hat das 1,6-GPS vor dem Vermischen mit dem Isomalt eine Partikelgröße von mindestens 30 µm. Erfindunggemäß bevorzugt hat das 1,6-GPS vor dem Vermischen mit dem Isomalt eine Partikelgröße von mindestens 40 µm. Erfindungsgemäß bevorzugt hat das 1,6-GPS vor dem Vermischen mit dem Isomalt eine Partikelgröße von mindestens 50 µm. Erfindungsgemäß bevorzugt hat das 1,6-GPS vor dem Vermischen mit dem Isomalt eine Partikelgröße von mindestens 60 µm. Erfindungsgemäß bevorzugt hat das 1,6-GPS vor dem Vermischen mit dem Isomalt eine Partikelgröße von mindestens 63 µm.

Erfindungsgemäß bevorzugt hat das 1,1-GPM vor dem Vermischen mit dem Isomalt eine Partikelgröße von höchstens 500 µm. Erfindungsgemäß bevorzugt hat das 1,1-GPM vor dem Vermischen mit dem Isomalt eine Partikelgröße von höchstens 250 µm. Erfindungsgemäß bevorzugt hat das 1,1-GPM vor dem Vermischen mit dem Isomalt eine Partikelgröße von höchstens 150 µm. Erfindungsgemäß bevorzugt hat das 1,1-GPM vor dem Vermischen mit dem Isomalt eine Partikelgröße von höchstens 100 µm. Erfindungsgemäß bevorzugt hat das 1,1-GPM vor dem Vermischen mit dem Isomalt eine Partikelgröße von 40 µm bis 150 µm. Erfindungsgemäß bevorzugt hat das 1,1-GPM vor dem Vermischen mit dem Isomalt eine Partikelgröße von 50 µm bis 100 µm. Erfindungsgemäß bevorzugt hat das das 1,1-GPM vor dem Vermischen mit dem Isomalt eine Partikelgröße von 63 µm bis 90 µm. Erfindungsgemäß bevorzugt hat das das 1,1-GPM vor dem Vermischen mit dem Isomalt eine Partikelgröße von mindestens 0,2 µm. Erfindungsgemäß bevorzugt hat das das 1,1-GPM vor dem Vermischen mit dem Isomalt eine Partikelgröße von mindestens 1 µm. Erfindungsgemäß bevorzugt hat das das 1,1-GPM vor dem Vermischen mit dem Isomalt eine Partikelgröße von mindestens 10 µm. Erfindungsgemäß bevorzugt hat das 1,1-GPM vor dem Vermischen mit dem Isomalt eine Partikelgröße von mindestens 20 µm. Erfindungsgemäß bevorzugt hat das 1,1-GPM vor dem Vermischen mit dem Isomalt eine Partikelgröße von mindestens 30 µm. Erfindungsgemäß bevorzugt hat das 1,1-GPM vor dem Vermischen mit dem Isomalt eine Partikelgröße von mindestens 40 µm. Erfindungsgemäß bevorzugt hat das 1,1-GPM vor dem Vermischen mit dem Isomalt eine Partikelgröße von mindestens 50 µm. Erfindungsgemäß bevorzugt hat das 1,1-GPM vor dem Vermischen mit dem Isomalt eine Partikelgröße von mindestens 60 µm. Erfindungsgemäß bevorzugt hat das 1,1-GPM vor dem Vermischen mit dem Isomalt eine Partikelgröße von mindestens 63 µm.

Erfindungsgemäß bevorzugt hat das 1,6-GPS oder das 1,1-GPM vor dem Vermischen mit dem Isomalt eine Reinheit von mindestens 90 Gew.-%, bevorzugt 98 Gew.-%, besonders bevorzugt 99 Gew.-%. Erfindungsgemäß bevorzugt hat das 1,6-GPS oder das 1,1-GPM vor dem Vermischen mit dem Isomalt eine Reinheit von mindestens 90 Gew.-%. Erfindungsgemäß bevorzugt hat das 1,6-GPS oder das 1,1-GPM vor dem Vermischen mit dem Isomalt eine Reinheit von mindestens 98 Gew.-%. Erfindungsgemäß bevorzugt hat das 1,6-GPS oder das 1,1-GPM vor dem Vermischen mit dem Isomalt eine Reinheit von mindestens 99 Gew.-%.

Erfindungsgemäß bevorzugt hat das 1,6-GPS vor dem Vermischen mit dem Isomalt eine Reinheit von mindestens 90 Gew.-%, bevorzugt 98 Gew.-%, besonders bevorzugt 99 Gew.-%. Erfindungsgemäß bevorzugt hat das 1,6-GPS vor dem Vermischen mit dem Isomalt eine Reinheit von mindestens 90 Gew.-%. Erfindungsgemäß bevorzugt hat das 1,6-GPS vor dem Vermischen mit dem Isomalt eine Reinheit von mindestens 98 Gew.-%. Erfindungsgemäß bevorzugt hat das 1,6-GPS vor dem Vermischen mit dem Isomalt eine Reinheit von mindestens 99 Gew.-%.

Erfindungsgemäß bevorzugt hat das 1,1-GPM vor dem Vermischen mit dem Isomalt eine Reinheit von mindestens 90 Gew.-%, bevorzugt 98 Gew.-%, besonders bevorzugt 99 Gew.-%. Erfindungsgemäß bevorzugt hat das 1,1-GPM vor dem Vermischen mit dem Isomalt eine Reinheit von mindestens 90 Gew.-%. Erfindungsgemäß bevorzugt hat das 1,1-GPM vor dem Vermischen mit dem Isomalt eine Reinheit von mindestens 98 Gew.-%. Erfindungsgemäß bevorzugt hat das 1,1-GPM vor dem Vermischen mit dem Isomalt eine Reinheit von mindestens 99 Gew.-%.

Erfindungsgemäß bevorzugt enthält die Mischung zusätzlich mindestens einen pharmazeutisch aktiven Wirkstoff. Im Zusammenhang mit der vorliegenden Erfindung werden unter pharmazeutisch aktiven Wirkstoffen Substanzen verstanden, die einen erwünschten prophylaktischen oder therapeutischen Effekt auf den menschlichen oder tierischen Körper haben. Diese Substanzen dienen also insbesondere der Prophylaxe und der Therapie von Mangelzuständen oder Krankheiten. Erfindungsgemäß bevorzugt sind beispielsweise Enzyme, Coenzyme, Mineralstoffe, Vitamine, Antibiotika, mikrobizid oder fungizid wirkende Stoffe oder andere pharmazeutisch wirksame Stoffe in der Mischung enthalten.

Erfindungsgemäß bevorzugt enthält die Mischung zusätzlich mindestens ein Fließmittel. Erfindungsgemäß bevorzugt beträgt der Fließmittelanteil der Mischung 0,1 bis 0,3 Gew.-%. Erfindungsgemäß bevorzugt ist das mindestens eine Fließmittel SiO₂. Erfindungsgemäß bevorzugt ist das mindestens eine Fließmittel Ca₃PO₄. Erfindungsgemäß bevorzugt enthält die Mischung zusätzlich mindestens ein Bindemittel. Erfindungsgemäß bevorzugt enthält die Mischung zusätzlich mindestens ein Schmiermittel. Erfindungsgemäß bevorzugt enthält die Mischung kein Fließmittel. Erfindungsgemäß bevorzugt enthält die Mischung kein Bindemittel. Erfindungsgemäß bevorzugt enthält die Mischung kein Schmiermittel.

Erfindungsgemäß bevorzugt enthält die Mischung zusätzlich Magnesiumstearat. Erfindungsgemäß bevorzugt enthält die Mischung weniger Magnesiumstearat als eine Mischung zur Herstellung eines vergleichbaren Komprimats aus dem Stand der Technik. Erfindungsgemäß bevorzugt enthält die Mischung kein Magnesiumstearat.

Erfindungsgemäß bevorzugt enthält die Mischung zusätzlich einen Stoff aus der Gruppe bestehend aus Aromastoffe, Farbstoffe, Sprengmittel, einen Intensiv-Süßstoff, Monosaccharide, Disaccharide, Monosaccharidalkohole, Disaccharidalkohole, Stärke, Stärkederivate, Cellulose, Cellulosederivate, Inulin oder Mischungen davon. Erfindungsgemäß bevorzugt enthält die Mischung zusätzlich mindestens einen Aromastoff. Erfindungsgemäß bevorzugt enthält die Mischung zusätzlich mindestens einen Farbstoff. Erfindungsgemäß bevorzugt enthält die Mischung zusätzlich mindestens ein Sprengmittel. Erfindungsgemäß bevorzugt enthält die Mischung zusätzlich mindestens einen Intensiv-Süßstoff. Erfindungsgemäß bevorzugt enthält die Mischung zusätzlich mindestens ein Monosaccharid. Erfindungsgemäß bevorzugt enthält die Mischung zusätzlich mindestens ein Disaccharid. Erfindungsgemäß bevorzugt enthält die Mischung zusätzlich mindestens ein Monosaccharidalkohol. Erfindungsgemäß bevorzugt enthält die Mischung zusätzlich mindestens ein Disaccharidalkohol. Erfindungsgemäß bevorzugt enthält die Mischung zusätzlich einen Stoff aus der Gruppe bestehend aus Stärke, Stärkederivate, Cellulose, Cellulosederivate, Inulin oder Mischungen davon.

Erfindungsgemäß bevorzugt enthält die Mischung kein Sprengmittel.

Erfindungsgemäß bevorzugt enthält die Mischung keinen Zucker. Erfindungsgemäß bevorzugt enthält die Mischung keine Saccharose. Erfindungsgemäß bevorzugt enthält die Mischung keine Glucose. Erfindungsgemäß bevorzugt enthält die Mischung keine Fructose.

Die Erfindung betrifft auch ein Komprimat, enthaltend a) Isomalt und zusätzlich b) 1,6-GPS (6-O-α-D-Glucopyranosyl-D-sorbit) oder 1,1-GPM (1-O-α-D-Glucopyranosyl-D-mannit).

Erfindungsgemäß bevorzugt handelt es sich bei dem Isomalt um ein weiter oben beschriebenes Isomalt. Erfindungsgemäß bevorzugt handelt es sich bei dem 1,6-GPS um ein weiter oben beschriebenes 1,6-GPS. Erfindungsgemäß bevorzugt handelt es sich bei dem 1,1-GPM um ein weiter oben beschriebenes 1,1-GPM.

Erfindungsgemäß bevorzugt enthält das Komprimat eine weiter oben beschriebene Mischung. Erfindungsgemäß bevorzugt besteht das Komprimat aus einer weiter oben beschriebenen Mischung.

Erfindungsgemäß bevorzugt enthält das Komprimat a) Isomalt und zusätzlich b) 1,6-GPS. Erfindungsgemäß bevorzugt enthält das Komprimat a) Isomalt und zusätzlich b) 1,1-GPM.

Erfindungsgemäß bevorzugt enthält das Komprimat zusätzlich einen pharmazeutisch aktiven Wirkstoff.

Erfindungsgemäß bevorzugt ist das Komprimat ein direkt verpresstes Komprimat.

Erfindungsgemäß bevorzugt enthält das Komprimat kein Magnesiumstearat.

Erfindungsgemäß bevorzugt enthält das Komprimat kein Sprengmittel.

Erfindungsgemäß bevorzugt enthält das Komprimat keinen Zucker. Erfindungsgemäß bevorzugt enthält das Komprimat keine Saccharose. Erfindungsgemäß bevorzugt enthält das Komprimat keine Glucose. Erfindungsgemäß bevorzugt enthält das Komprimat keine Fructose.

Erfindungsgemäß bevorzugt ist das Komprimat der Kern eines Dragees.

Erfindungsgemäß bevorzugt ist das Komprimat nicht mit einer umhüllenden Schicht überzogen. Erfindungsgemäß bevorzugt ist das Komprimat mit einer umhüllenden Schicht überzogen.

Erfindungsgemäß bevorzugt wird das Komprimat nach einem erfindungsgemäßen Verfahren hergestellt.

Erfindungsgemäß bevorzugt hat das Komprimat eine kurze Auflösedauer. Erfindungsgemäß bevorzugt hat das Komprimat eine kürzere Auflösedauer als ein vergleichbares Komprimat aus dem Stand der Technik. Erfindungsgemäß bevorzugt hat das Komprimat eine Auflösedauer von 500 Sekunden bis 5 Sekunden. Erfindungsgemäß bevorzugt hat das Komprimat eine Auflösedauer von 200 Sekunden bis 10 Sekunden. Erfindungsgemäß bevorzugt hat das Komprimat eine Auflösedauer von unter 200 Sekunden. Erfindungsgemäß bevorzugt hat das Komprimat eine Auflösedauer von unter 100 Sekunden. Erfindungsgemäß bevorzugt hat das Komprimat eine Auflösedauer von unter 60 Sekunden.

Im Zusammenhang mit der vorliegenden Erfindung wird unter Auflösedauer eines Komprimats die Zerfallszeit verstanden. Die Auflösedauer kann zum Beispiel nach dem Verfahren zur Bestimmung der Zerfallszeit nach Ph. Eur. 2.9.1 des Europäischen Arzneibuchs bestimmt werden.

Ein Fachmann kennt die für seine Zwecke geeignete Auflösedauer eines Komprimats.

Die Erfindung betrifft auch eine Mischung aus Isomalt und 1,6-GPS oder 1,1-GPM. Besonders bevorzugte Ausführungsformen einer erfindungsgemäßen Mischung und ihrer Bestandteile sind weiter oben beschrieben.

Die Erfindung betrifft auch die Verwendung einer erfindungsgemäßen Mischung zur Herstellung eines Komprimates.

Weitere vorteilhafte Ausgestaltungen der Erfindung finden sich in den Unteransprüchen.

Die folgenden Beispiele und die Figuren erläutern die Erfindung in Einzelheiten.

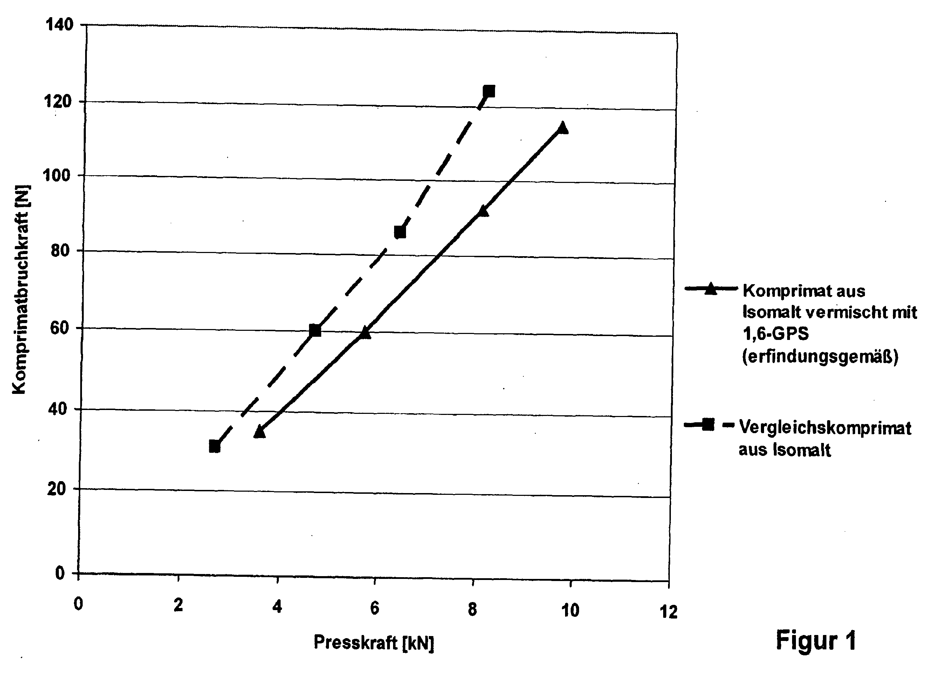

Figur 1 zeigt das Verhältnis von Presskraft zu Komprimathärte (gemessen als Komprimatbruchkraft) von erfindungsgemäßen Komprimaten, die aus einer Mischung aus Isomalt und 1,6-GPS mit verschiedenen Presskräften hergestellt wurden, und vergleichbaren Komprimaten, die aus einer Isomalt-Mischung ohne Zusatz von 1,6-GPS hergestellt wurden. Die erfindungsgemäßen Komprimate und die Vergleichskomprimate enthalten 1,6-GPS und 1,1-GPM im gleichen Mengenverhältnis von 3 : 1.

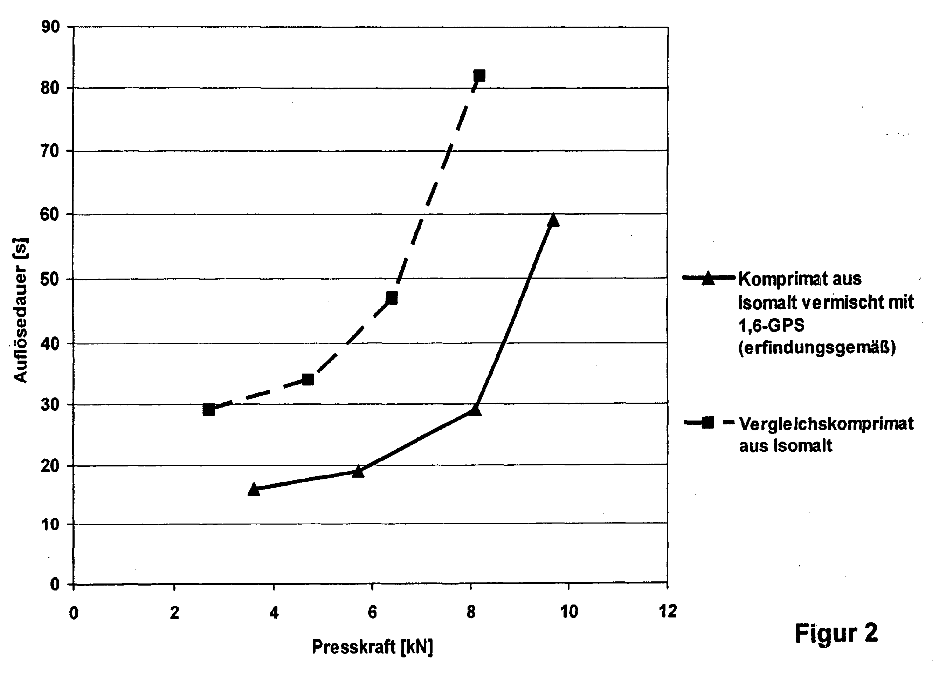

Figur 2 zeigt das Verhältnis von Presskraft zu Auflösedauer von erfindungsgemäßen Komprimaten, die aus einer Mischung aus Isomalt und 1,6-GPS mit verschiedenen Presskräften hergestellt wurden, und vergleichbaren Komprimaten, die aus einer Isomalt-Mischung ohne Zusatz von 1,6-GPS hergestellt wurden. Die erfindungsgemäßen Komprimate und die Vergleichskomprimate enthalten 1,6-GPS und 1,1-GPM im gleichen Mengenverhältnis von 3 : 1.

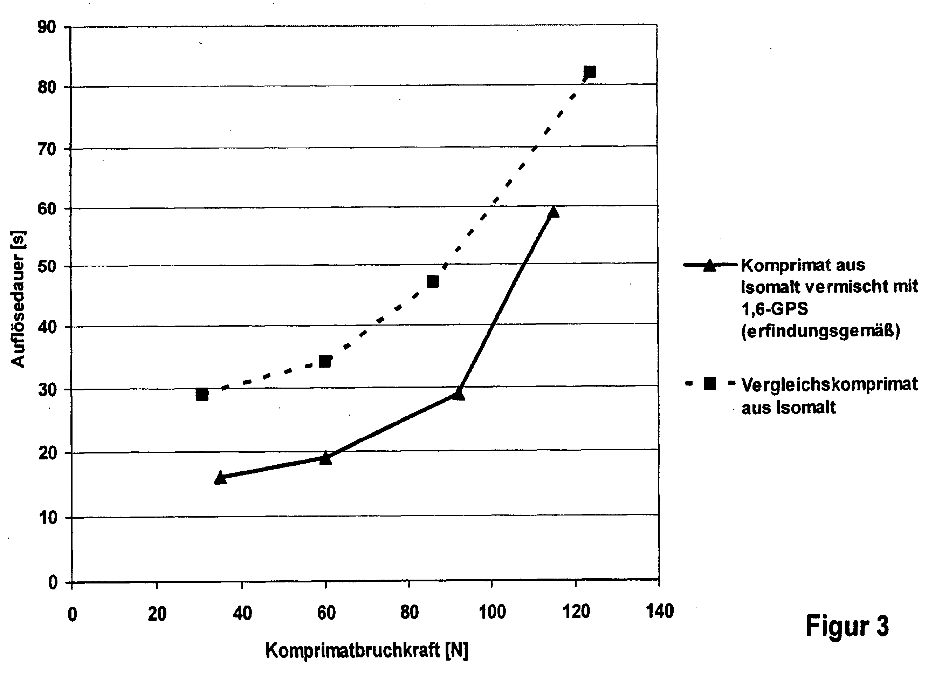

Figur 3 zeigt das Verhältnis von Komprimathärte (gemessen als Komprimatbruchkraft) zu Auflösedauer von erfindungsgemäßen Komprimaten, die aus einer Mischung aus Isomalt und 1,6-GPS mit verschiedenen Presskräften hergestellt wurden, und vergleichbaren Komprimaten, die aus einer Isomalt-Mischung ohne Zusatz von 1,6-GPS hergestellt wurden. Die erfindungsgemäßen Komprimate und die Vergleichskomprimate enthalten 1,6-GPS und 1,1-GPM im gleichen Mengenverhältnis von 3 : 1.

### Beispiele:

### Beispiel 1: Herstellung erfindungsgemäßer Komprimate aus Isomalt und 1,6-GPS

1,6-GPS wurde so gemahlen, dass 5 Gew.-% des 1,6-GPS eine Partikelgröße von mindestens 125,98 µm, 50 Gew.-% des 1,6-GPS eine Partikelgröße von mindestens 42,18 µm und 95 Gew.-% des 1,6-GPS eine Partikelgröße von mindestens 4,11 µm hatten. Danach wurde das 1,6-GPS gesiebt, so dass das gesamte 1,6-GPS kleiner als 100 µm war.

Als Isomalt wurden zwei verschiede Isomalt-Sorten verwendet.

Sorte A enthielt 53,55 Gew.-% 1,6-GPS und 45,69 Gew.-% 1,1-GPM. 11,8 Gew.-% hatten eine Korngröße von kleiner 63 µm. 88,12 Gew.-% hatten eine Korngröße von 63 µm bis 500 µm. 0,08 Gew.-% hatten eine Korngröße von über 500 µm.

Sorte B enthielt 75,93 Gew.-% 1,6-GPS und 22,63 Gew.-% 1,1-GPM. 8,34 Gew.-% hatten eine Korngröße von kleiner 63 µm. 91,52 Gew.-% hatten eine Korngröße von 63 µm bis 500 µm. 0,14 Gew.-% hatten eine Korngröße von über 500 µm.

Die beiden Isomalt-Sorten wurden jeweils mit 10 Gew.-%, 30 Gew.-%, 50 Gew.-% und 90 Gew.-% (bezogen auf die Gesamtmenge an Isomalt und zusätzlichem 1,6-GPS) 1,6-GPS in einem Diffusionsmischer vermischt.

Aus der Mischung wurden Komprimate mit einem Soll-Gewicht von 600 mg und einem Soll-Durchmesser von 12 mm mit einer Presse der Bauart FETTE P1200 IG direkt gepresst. Dabei wurden verschiedene Presskräfte angesetzt, so dass Komprimate mit einer Komprimathärte von etwa 30 N, 60 N, 90 N oder 120 N hergestellt wurden. Von jeder Komprimatsorte wurden mindestens 16 Komprimate hergestellt.

### Beispiel 2: Herstellung erfindungsgemäßer Komprimate aus Isomalt und 1,1-GPM

1,1-GPM wurde so gemahlen, dass 5 Gew.-% des 1,1-GPM eine Partikelgröße von mindestens 67,48 µm, 50 Gew.-% des 1,1-GPM eine Partikelgröße von mindestens 17,71 µm und 95 Gew.-% des 1,1-GPM eine Partikelgröße von mindestens 1,11 µm hatten. Danach wurde das 1,1-GPM gesiebt, so dass das gesamte 1,1-GPM kleiner als 100 µm war.

Als Isomalt wurden die zwei verschiede Isomalt-Sorten von Beispiel 1 verwendet.

Die beiden Isomalt-Sorten wurden jeweils mit 10 Gew.-%, 30 Gew.-%, 50 Gew.-% und 90 Gew.-% (bezogen auf die Gesamtmenge an Isomalt und zusätzlichem 1,1-GPM) 1,1-GPM wie in Beispiel 1 vermischt.

Aus der Mischung wurden Komprimate mit einem Soll-Gewicht von 600 mg und einem Soll-Durchmesser von 12 mm mit einer Presse der Bauart FETTE P1200 IG direkt gepresst. Dabei wurden verschiedene Presskräfte angesetzt, so dass Komprimate mit einer Komprimathärte von etwa 30 N, 60 N, 90 N oder 120 N hergestellt wurden. Von jeder Komprimatsorte wurden mindestens 16 Komprimate hergestellt.

### Beispiel 3: Messung der Bruchkraft

Zur Messung der Bruchkraft wurde die Komprimathärte nach der in der Ph. Eur. unter 2.9.8 beschriebenen Methode bestimmt. Die Durchführung der Messung erfolgte mit dem Messgerät TBH 30 der Firma Erweka.

Es wurde die Bruchkraft von jeweils vier erfindungsgemäßen Komprimaten nach Beispiel 1 aus der Isomalt-Sorte A und 50 Gew.-% 1,6-GPS und von jeweils vier Komprimaten aus einer 1,6-GPS angereicherten Isomalt-Mischung ohne Zusatz von 1,6-GPS gemessen. Die erfindungsgemäßen Komprimate und die Vergleichskomprimate enthielten 1,6-GPS und 1,1-GPM im gleichen Mengenverhältnis.

Der Vergleich der Bruchkraft der erfindungsgemäßen Komprimate und der Vergleichskomprimate ist Figur 1 zu entnehmen.

### Beispiel 4: Messung der Auflösedauer

Die Auflösedauer wurde mittels eines Zerfallszeittesters gemessen.

Es wurde die Auflösedauer von jeweils vier erfindungsgemäßen Komprimaten nach Beispiel 1 aus der Isomalt-Sorte A und 50 Gew.-% 1,6-GPS und von jeweils vier Komprimaten aus einer 1,6-GPS angereicherten Isomalt-Mischung ohne Zusatz von 1,6-GPS gemessen. Die erfindungsgemäßen Komprimate und die Vergleichskomprimate enthielten 1,6-GPS und 1,1-GPM im gleichen Mengenverhältnis.

Der Vergleich der Auflösedauer der erfindungsgemäßen Komprimate und der Vergleichskomprimate ist Figur 2 zu entnehmen.

Aus Figur 3 ergibt sich bei gleicher Härte eine geringere Auflösedauer der erfindungsgemäßen Komprimate im Vergleich zu reinen Isomalt-Komprimaten mit gleichem Anteil an 1,6-GPS und 1,1-GPM.

### Beispiel 5: Kristallstrukturaufnahmen mit Raster-Elektronen-Mikroskop

Bei einem Vergleich von Raster-Elektronen-Mikroskop (REM)-Aufnahmen der Bruchflächen von erfindungsgemäßen Komprimaten und Bruchflächen von Vergleichskomprimaten ist bei den erfindungsgemäßen Komprimaten ein offenporigeres Gefüge mit größerer Porenanzahl zu erkennen.

## Patentansprüche

1. Verfahren zur Herstellung eines Komprimates, wobei Isomalt mit 1,6-GPS (6-O-α-D-Glucopyranosyl-D-sorbit) oder mit 1,1-GPM (1-O-α-D-Glucopyranosyl-D-mannit) vermischt wird und die Mischung zu einem Komprimat verpresst wird.

2. Verfahren nach Anspruch 1, wobei Isomalt mit 1,6-GPS vermischt wird und die Mischung zu einem Komprimat verpresst wird.

3. Verfahren nach Anspruch 1, wobei Isomalt mit 1,1-GPM vermischt wird und die Mischung zu einem Komprimat verpresst wird.

4. Verfahren nach einem der vorhergehenden Ansprüche, wobei das Verhältnis zwischen Isomalt und 1,6-GPS oder 1,1-GPM 1 : 9 bis 9 : 1 beträgt.

5. Verfahren nach einem der vorhergehenden Ansprüche, wobei das Verhältnis zwischen Isomalt und 1,6-GPS oder 1,1-GPM 3 : 7 bis 7 : 3 beträgt.

6. Verfahren nach einem der vorhergehenden Ansprüche, wobei das Isomalt 43 Gew.-% bis 80 Gew.-% 1,6-GPS enthält.

7. Verfahren nach einem der vorhergehenden Ansprüche, wobei das 1,6-GPS oder das 1,1-GPM vor dem Vermischen mit dem Isomalt gemahlen wird.

8. Verfahren nach einem der vorhergehenden Ansprüche, wobei das 1,6-GPS oder das 1,1-GPM vor dem Vermischen mit dem Isomalt eine Partikelgröße von höchstens 100 µm hat.

9. Komprimat, enthaltend
a) Isomalt und zusätzlich
b) 1,6-GPS (6-O-α-D-Glucopyranosyl-D-sorbit) oder 1,1-GPM (1-O-α-D-Glucopyranosyl-D-mannit).

10. Komprimat nach Anspruch 9, enthaltend
a) Isomalt und zusätzlich
b) 1,6-GPS.

11. Komprimat nach Anspruch 9, enthaltend
a) Isomalt und zusätzlich
b) 1,1-GPM.

12. Mischung aus Isomalt und 1,1-GPM.

13. Verwendung einer Mischung aus Isomalt und 1,6-GPS oder Isomalt und 1,1-GPM zur Herstellung eines Komprimates.

14. Verwendung einer Mischung aus Isomalt und 1,6-GPS oder Isomalt und 1,1-GPM bei der Herstellung eines Komprimates zur Regulierung der Komprimat-Auflösedauer.

15. Verwendung einer Mischung aus Isomalt und 1,6-GPS oder Isomalt und 1,1-GPM bei der Herstellung eines Komprimates zur Regulierung der Komprimathärte.

## Claims

1. A method for producing a tablet, wherein isomalt is mixed with 1,6-GPS (6-O-α-D-Glucopyranosyl-D-sorbitol) or with 1,1-GPM (1-O-α-D-Glucopyranosyl-D-mannitol) and the mixture is pressed into a tablet.

2. The method according to claim 1, wherein isomalt is mixed with 1,6-GPS and the mixture is pressed into a tablet.

3. The method according to claim 1, wherein isomalt is mixed with 1,1-GPM and the mixture is pressed into a tablet.

4. The method according to any one of the preceding claims, wherein the ratio between isomalt and 1,6-GPS or 1,1-GPM is 1 : 9 to 9 : 1.

5. The method according to any one of the preceding claims, wherein the ratio between isomalt and 1,6-GPS or 1,1-GPM is 3 : 7 to 7 : 3.

6. The method according to any one of the preceding claims, wherein the isomalt contains 43% by weight to 80% by weight 1,6-GPS.

7. The method according to any one of the preceding claims, wherein the 1,6-GPS or the 1,1-GPM are ground prior to mixing with the isomalt.

8. The method according to any one of the preceding claims, wherein the 1,6-GPS or the 1,1-GPM have a particle size of at most 100 µm prior to mixing with the isomalt.

9. A tablet, containing
a) isomalt and, in addition
b) 1,6-GPS (6-O-α-D-Glucopyranosyl-D-sorbitol) or 1,1-GPM (1-O-α-D-Glucopyranosyl-D-mannitol).

10. The tablet according to claim 9, containing
a) isomalt and, in addition
b) 1,6-GPS.

11. The tablet according to claim 9, containing
a) isomalt and, in addition
b) 1,1-GPM.

12. A mixture of isomalt and 1,1-GPM.

13. A use of a mixture of isomalt and 1,6-GPS or isomalt and 1,1-GPM for producing a tablet.

14. The use of a mixture of isomalt and 1,6-GPS or isomalt and 1,1-GPM in the production of a tablet for controlling the tablet dissolution period.

15. The use of a mixture of isomalt and 1,6-GPS or isomalt and 1,1-GPM in the production of a tablet for controlling the tablet hardness.

## Revendications

1. Procédé pour la fabrication d'un comprimé, dans lequel de l'isomalt est mélangé avec 1,6-GPS (6-O-α-D-glucopyranosyl-D-sorbitol) ou avec 1,1-GPM (1-O-α-D-glucopyranosyl-D-mannitol) et le mélange est soumis à un pressage pour former un comprimé.

2. Procédé selon la revendication 1, dans lequel l'isomalt est mélange avec 1,6-GPS et le mélange est soumis à un pressage pour former un comprimé.

3. Procédé selon la revendication 1, dans lequel l'isomalt est mélange avec 1,1-GPM et le mélange est soumis à un pressage pour former un comprimé.

4. Procédé selon l'une quelconque des revendications précédentes, dans lequel le rapport de l'isomalt et du 1,6-GPS ou 1,1-GPM est de 1 : 9 à 9 : 1.

5. Procédé selon l'une quelconque des revendications précédentes, dans lequel le rapport de l'isomalt et du 1,6-GPS ou 1,1-GPM est de 3 : 7 à 7 : 3.

6. Procédé selon l'une quelconque des revendications précédentes, dans lequel fisomalt contient de 43 % en poids à 80 % en poids du 1,6-GPS.

7. Procédé selon l'une quelconque des revendications précédentes, dans lequel le 1,6-GPS ou le 1,1-GPM est moulu avant le mélange avec l'isomalt.

8. Procédé selon l'une quelconque des revendications précédentes, dans lequel le 1,6-GPS ou le 1,1-GPM présente une granulométrie n'excédant 100 µm avant le mélange avec l'isomalt.

9. Comprimé contenant
a) de l'isomalt et en plus
b) 1,6-GPS (6-O-α-D-glucopyranosyl-D-sorbitol) ou 1,1-GPM (1-O-α-D-glucopyranosyl-D-mannitol).

10. Comprimé selon la revendication 9, contenant
a) de l'isomalt et en plus
b) 1,6-GPS.

11. Comprimé selon la revendication 9, contenant
a) de l'isomalt et en plus
b) 1,1-GPM.

12. Mélange d'isomalt et 1,1-GPM.

13. Utilisation d'un mélange de l'isomalt et 1,6-GPS ou de l'isomalt et 1,1-GPM pour la fabrication d'un comprimé.

14. Utilisation d'un mélange de l'isomalt et 1,6-GPS ou de l'isomalt et 1,1-GPM dans la fabrication d'un comprimé pour contrôler la durée de dissolution du comprimé.

15. Utilisation d'un mélange de l'isomalt et 1,6-GPS ou de l'isomalt et 1,1-GPM dans la fabrication d'un comprimé pour contrôler la dureté du comprimé.
